# NOUVEAU FASCICULE DE BREVET EUROPEEN

(11) **EP 0 154 756 B2**
(45) Date de publication et mention de la décision concernant l'opposition: **23.10.1996**
(45) Mention de la délivrance du brevet: 16.08.1989
(21) Numéro de dépôt: 84401984.4
(22) Date de dépôt: 04.10.1984
(51) Int. Cl.: C07D 461/00, A61K 31/475

(54) **Citrate de vinpocetine et son procédé de préparation**
Vinpocetin-Citrate und Verfahren zu seiner Herstellung
Citrate of vinpocetine, and process for its preparation

(30) Priorité: 29.02.1984 ES 530165; 21.03.1984 ES 530837
(43) Date de publication de la demande: 18.09.1985
(73) Titulaire: COVEX (S.A.), E-28016 Madrid (ES)
(72) Inventeur: Manresa, Maria Teresa, 28018 Madrid (ES); Calvo, Fernando, 28034 Madrid (ES)
(74) Mandataire: VOSSIUS & PARTNER

(56) Documents cités:
- AT-A- 377 917
- DE-A- 2 253 750
- FR-A- 2 190 433
- FR-A- 2 191 891
- FR-A- 2 191 894
- FR-A- 2 228 479
- FR-A- 2 283 669
- FR-A- 2 468 605
- GB-A- 1 405 127
- US-A- 4 400 514
- CHEMICAL ABSTRACTS, volume 99, 1983, page 678, abrégé 122742h, Columbus, Ohio, US
- J.G. Wagner "Fundamentals of Clinical Pharmacokinetics", 1975, Drug Intelligence Publications Inc., Hamilton US, pp. 11-14

## Description

La présente invention concerne le citrate de (+) vinpocetine et la préparation de ce sel.

Le citrate de (+) vinpocetine répond à la formule (I) suivante: 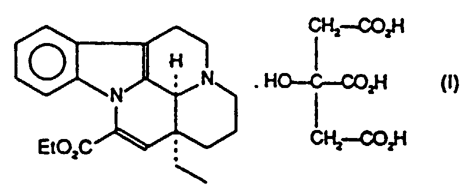 dans laquelle Et désigne le radical éthyle.

La vinpocetine [(3α, 16α) - éburnamenine-14-carboxylate d'éthyle)] est un alcaloïde de la série de l'éburnane, obtenue par semi-synthèse à partir de la vincamine (brevet espagnol n° 408 180, brevet français n° 2 468 605). La vinpocetine présente une série de propriétés pharmacologiques intéressantes en relation avec la circulation cérébrale, en agissant sur la résistance vasculaire, particulièrement dans la région des vaisseaux cérébraux (Drug. Res. 10 26, 1907, [1976]).

Sous l'effet de la vinpocetine, la pression sanguine et l'activité cardiaque décroissent légèrement, c'est pourquoi elle s'applique aux maladies cérébrales accompagnées d'hypertension. La vinpocetine améliore l'oxygénation cérébrale et la tolérance à l'anoxie des cellules cérébrales. Par son effet biochimique, elle augmente la concentration de l'acide AMP, de la sérotonine et de l'acide ATP dans les tissus, favorisant la fonction cérébrale. Ainsi, elle augmente le métabolisme aérobie et anaérobie du glucose.

On connaît déjà, outre la vinpocétine, des alcaloïdes favorisant la circulation sanguine dans le cerveau.

US-A 4 400 514 décrit un procédé de préparation de dérivés de l'ester acide apovincaminique comprenant deux radicaux alkyles en hydrogénant l'un des composants. Après quoi, des composés acceptables d'un point de vue pharmaceutique peuvent être obtenus par action d'un acide inorganique ou organique.

FR-A 2 283 669 décrit le 2-cétoglutamate de vincamine et son procédé de préparation. Selon ce procédé, on mélange dans un solvant de l'acide 2-cétoglutarique et de la vincamine, puis on fait précipiter le 2-cétoglutarate à partir du mélange ainsi formé.

C'est ce dernier point qui a conduit la Demanderesse à l'idée de préparer un sel de vinpocetine qui améliore l'action de celle-ci et qui facilite son absorption. On a réalisé une série de sels à partir des acides qui interviennent dans le métabolisme du glucose, parmi lesquels on a isolé le citrate de vinpocetine pour ses propriétés pharmacologiques, pour sa rapide absorption par l'organisme et pour sa plus grande solubilité en solution aqueuse, ce qui permet de réaliser des préparations par voie orale, en gouttes ou injectables.

Les propriétés pharmacologiques du citrate de vinpocetine ont été utilisées en thérapie avec de bons résultats. Le tableau ci-après rassemble les résultats obtenus avec le citrate de vinpocetine.

| Composant | DL50 | Effet Hypotension | Cerveau | | | |
|---|---|---|---|---|---|---|
| | | | Augmentation circulation | | Diminution résistance vasculaire | |
| | | | i.v. | i.s. | i.v. | i.s. |
| Vinpocetine | 1,0 | 1,0 | 1,0 | 1,0 | 1,0 | 1,0 |
| Citrate de vinpocetine | 1,5 | 0,8 | 0,8 | 0,7 | 0,9 | 0,6 |

Dans l'étude de l'effet de ce composé, on a enregistré et calculé le débit sanguin cérébral (artère carotide interne) et la résistance vasculaire. Les composés ont été administrés par voie intraveineuse et intraartérielle sur des chiens dont on a déterminé les valeurs de DLSO. Les résultats figurant dans le tableau précité sont rapportés à la vinpocetine à laquelle on a donné une valeur arbitraire de 1,0, c'est-à-dire que la valeur relative obtenue pour le citrate (I) est élevée, alors que sa toxicité est basse. Pour l'étude des effets sur la pression sanguine et la circulation cérébrale, on a administré des doses intraveineuses de 1 à 4 mg/kg d'animal.

Les résultats obtenus montrent que le citrate de vinpocetine produit une augmentation effective de la circulation cérébrale et présente une toxicité nettement diminuée par rapport à la vinpocetine base, le citrate de vinpocetine étant une fois et demie moins toxique que la vinpocetine base.

Par ailleurs, alors que la vinpocetine est un produit insoluble dans l'eau, ce qui permet difficilement d'en préparer des solutions aqueuses utilisables par voie orale, en gouttes ou injectables, le citrate de vinpocetine (I) est soluble en solution aqueuse, ce qui permet son utilisation par voie orale.

Le procédé de préparation du citrate de vinpocetine (I) conforme à l'invention est caractérisé en ce que l'on fait réagir la (+) vinpocetine avec une solution de l'éthanol absolu de citrique. Selon ledit procédé, on maintient la solution réactionnelle à une température de l'ordre de 20°C pendant une durée de 5 à 10 minutes, en agitant énergiquement Pendant ce laps de temps la solution se concentre à la moitié de son volume initial et l'on obtient, après refroidissement à une température de l'ordre de 0°C, un précipité de citrate de vinpocetine qui est lavé et laissé sécher à l'air.

De la même façon, le citrate de vinpocetine (I) peut être préparé à partir d'autres sels de (+) vinpocetine, tel que le phosphate obtenu selon le procédé précédemment décrit, en le faisant réagir avec le sel sodique ou potassique de l'acide citrique.

L'avantage de ces procédés réside dans le fait qu'ils permettent de préparer d'une manière simple, peu coûteuse et avec de bons rendements, le citrate de (+) vinpocetine (I) à des fins thérapeutiques.

D'autres avantages et caractéristiques apparaîtront à la lecture de l'exemple de réalisation donné ci-après, à titre purement illustratif et non limitatif.

### Exemple

2 g d'acide citrique sont dissous dans 50 ml d'éthanol absolu et on ajoute à cette solution 2 g de (+) vinpocetine. Le mélange est agité pendant 10 minutes au bout desquelles la solution se concentre à la moitié de son volume initial et on refroidit ladite solution à 0°C, ce qui provoque la formation d'un précipité de citrate, qu'on lave avec 2 ml d'alcool absolu, et qu'on laisse sécher à l'air, en l'absence de lumière. Le produit est le citrate de (+) vinpocetine (I) présentant un point de fusion de 194-195°C (rendement 90%).

## Revendications

1. Sel de (+) vinpocetine présentant une solubilité dans l'eau améliorée et notamment destiné à des fins thérapeutiques, caractérisé en ce qu'il répond à la formule (I) suivante: 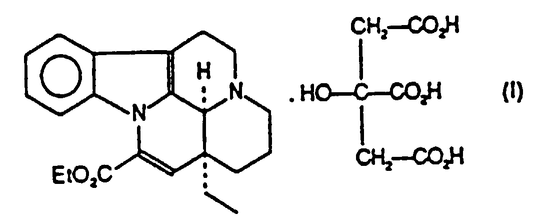 dans laquelle Et désigne le radical éthyle.

2. Procédé de préparation du citrate de (+) vinpocetine de formule (I), caractérisé en ce que l'on fait réagir en milieu de l'éthanol absolu la vinpocetine avec l'acide citrique à une température de l'ordre de 20°C pendant une durée de 5 à 10 minutes pour obtenir le citrate de (+) vinpocetine (I), et le produit final est obtenu par concentration de la solution réactionnelle à la moitié de son volume initial et par refroidissement à une temperature de l'ordre de 0°C, provoquant la formation d'un précipité correspondant au sel recherché.

3. Procédé de préparation du citrate de (+) vinpocetine de formule (I), caractérisé en ce que l'on fait réagir en milieu de l'éthanol absolu un sel de (+) vinpocetine, tel que le phosphate avec du citrate sodique ou potassique, à une température de 20°C pendant une durée de 5 à 10 minutes, pour obtenir le citrate de (+) vinpocetine, et le produit final est obtenu par concentration de la solution réactionnelle à la moitié de son volume initial et par refroidissement à une temperature de l'ordre de 0°C, provoquant la formation d'un précipité correspondant au sel recherché.

## Patentansprüche

1. Salz von (+) Vinpocetin mit verbesserter Wasserlöslichkeit und insbesondere für therapeutische Zwecke bestimmt, dadurch gekennzeichnet, daß es folgender Formel (I) entspricht: 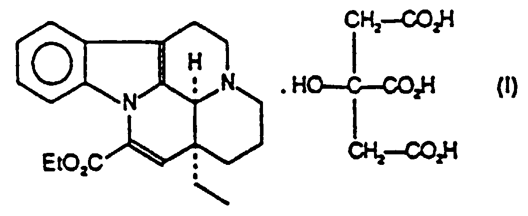 in welcher Et die Ethylgruppe bezeichnet.

2. Verfahren zum Herstellen von (+) Vinpocetin-Zitrat gemäß Formel (I), dadurch gekennzeichnet, daß man das Vinpocetin in reinem Ethanol als Medium mit Zitronensäure bei einer Temperatur in der Größenordnung von 20°C während einer Dauer von 5 bis 10 Minuten reagieren läßt, um das (+) Vinpocetin-Zitrat (I) zu erhalten, und das Endprodukt durch Reduzierung der Reaktionslösung auf die Hälfte ihres Ausgangsvolumens und durch Abkühlen auf eine Temperatur in der Größenordnung von 0°C erhalten wird, wobei die Bildung eines Niederschlags hervorgerufen wird, der dem gesuchten Salz entspricht.

3. Verfahren zum Herstellen von (+) Vinpocetin-Zitrat gemäß Formel (I), dadurch gekennzeichnet, daß man ein Salz des (+) Vinpocetins, wie das Phosphat, in reinem Ethanol als Medium mit Natrium- oder Kaliumzitrat bei einer Temperatur von 20°C während einer Dauer von 5 bis 10 Minuten reagieren läßt, um das (+) Vinpocetin-Zitrat zu erhalten, und das Endprodukt durch Reduzierung der Reaktionslösung auf die Hälfte ihres Ausgangsvolumens und durch Abkühlen auf eine Temperatur in der Größenordnung von 0°C erhalten wird, wobei die Bildung eines Niederschlags hervorgerufen wird, der dem gesuchten Salz entspricht.

## Claims

1. A salt of (+) vinpocetine having improved solubility in water, and intended particularly for therapeutic purposes, characterized in that it has the following formula (I): 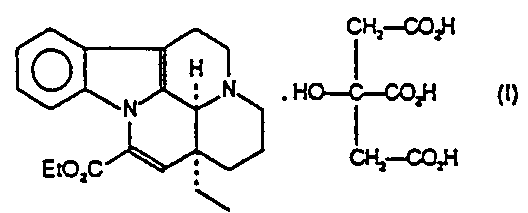 in which Et designates the ethyl radical.

2. A method of preparing (+) vinpocetine citrate according to the formula (I), characterized in that the vinpocetine is caused to react with citric acid in pure ethanol as medium at a temperature of about 20°C for 5 to 10 minutes in order to obtain the (+) vinpocetine citrate (I), and the final product is obtained by concentrating the reaction solution to one half of its initial volume and by cooling the said solution to a temperature of about 0°C, thereby causing a precipitate corresponding to the desired salt to be formed.

3. A method of preparing (+) vinpocetine citrate according to the formula (I), characterized in that a salt of (+) vinpocetine, such as vinpocetine phosphate, is caused to react with sodium or potassium citrate in pure ethanol as medium at a temperature of 20°C for 5 to 10 minutes in order to obtain the (+) vinpocetine citrate (I), and the final product is obtained by concentrating the reaction solution to one half of its initial volume and by cooling the said solution to a temperature of about 0°C, thereby causing a precipitate corresponding to the desired salt to be formed.
